# FASCICULE DE BREVET EUROPEEN

(11) **EP 3 335 650 B1**
(45) Date de publication et mention de la délivrance du brevet: **21.08.2019**
(21) Numéro de dépôt: 17204286.3
(22) Date de dépôt: 05.06.2014
(51) Int. Cl.: A61B 17/34, A61B 17/22, A61B 10/02

(54) **TROCART PERFORANT**
TROKAR ZUM DURCHSTECHEN
PERFORATING TROCAR

(30) Priorité: 01.07.2013 FR 1356391
(43) Date de publication de la demande: 20.06.2018
(62) Demande divisionnaire de: 14733269.6
(73) Titulaire: Merit Medical Systems, Inc., South Jordan, UT 84095 (US)
(72) Inventeur: Fumex, Laurent, Madison, CT 06443 (US); Masseglia, Thierry, 83130 La Garde (FR)
(74) Mandataire: CSY London

(56) Documents cités:
- WO-A1-2006/061514
- DE-A1- 3 542 948
- US-A1- 2003 212 343
- US-A1- 2003 225 411
- US-A1- 2009 204 024
- US-B1- 6 575 919
- US-B2- 7 850 620

## Description

La présente invention concerne un dispositif utilisable en chirurgie et en radiologie interventionnelle, et plus particulièrement un trocart perforant utilisable notamment dans le domaine des procédures percutanées de la biopsie osseuse ou de moelle, de la vertébroplastie, de la cimentoplastie des régions squelettiques, et plus généralement du traitement des lésions osseuses.

On connaît divers types de trocarts perforants qui sont des instruments chirurgicaux utilisés pour forer l'os de façon à atteindre une zone où la biopsie osseuse doit être effectuée. Ces trocarts sont composés d'un tube externe creux, dont l'extrémité est plus ou moins coupante, et d'une tige, dont l'extrémité est affûtée de façon à perforer l'os, coulissant dans le tube.

Ainsi, la demande de brevet WO 2006/061514 décrit un trocart destiné à la biopsie osseuse comprenant un tube externe, dont l'extrémité distale est divisée en deux segments à bord coupant hélicoïdal, dans lequel coulisse une tige affûtée. Ce type d'instrument est utilisé manuellement au moyen d'une poignée.

Le brevet US 7850620 décrit un trocart destiné à la biopsie de moelle osseuse composé d'un tube externe, dont l'extrémité distale comporte un affûtage traditionnel pour ce type d'instrument, associé à une tige affûtée de façon traditionnelle. Cet instrument est utilisé en l'accouplant à une perceuse.

Les demandes de brevet US 2003/225411 A1 et US 2009/0204024 A1 concernent des trocarts destinés à la biopsie de moelle osseuse. Ces trocarts comportent chacun une tige ayant une encoche permettant l'évacuation de morceaux d'os et de tissu. Le brevet US 6,575,919 B1 décrit un trocart ayant une ouverture intérieure pour permettre le passage d'une aiguille.

Les trocarts perforants connus permettent de forer l'os mais ne peuvent pas être guidés sur broche en même temps. Cette lacune présente deux inconvénients majeurs: l'absence de précision au moment de l'abord osseux et le risque d'accident. Or, les procédures percutanées sont essentiellement effectuées sous imagerie et donc à partir d'images. Le point d'entrée et la trajectoire peuvent ainsi être visualisés et définis à l'avance. L'importance de la précision du point d'entrée se comprend parfaitement en cas de petites lésions osseuses car celles-ci ont des diamètres ne dépassant parfois pas le millimetre; la difficulté est naturellement accrue lorsqu'il s'agit d'atteindre une lésion trouvant son siège dans un os profond . Ainsi il n'est pas rare chez un patient obèse d'avoir à traverser 150mm à 200mm de parties molles avant d'atteindre la corticale osseuse. Maintenir dans de telles conditions au millimètre près la trajectoire du trocart dont le diamètre est d'environ 2mm est particulièrement difficile. De plus, il y a des risques liés à une introduction directe d'un trocart perforant lorsque cet os est situé dans une zone dense comprenant des organes, des veines et des nerfs. Confronté à de telles situations, le praticien acceptera de multiplier les gestes et placera d'abord manuellement une broche rigide fine et peu invasive, laquelle servira de guide aux divers trocarts et instruments coaxiaux pour la suite de la procédure opératoire.

On connaît également des mèches perforées, utilisées en orthopédie, qui permettent de forer l'os en étant guidées sur broche. Cependant, ces mèches ne sont pas ou peu employées dans le cadre d'une procédure percutanée et encore moins lorsque celle-ci est effectuée manuellement. L'utilisation de ces mèches exige que la broche soit d'abord fichée dans l'os au moyen d'une perceuse ; en effet, ces mèches n'ont pas de pointe distale étant perforées de part en part afin de coulisser sur la broche. Elles risquent donc, en l'absence de broche fichée, de glisser sur l'os et de dévier du point d'entrée défini par le praticien.

La présente invention a pour objet un dispositif, notamment pour la chirurgie et la radiologie interventionnelle, procurant une bonne efficacité de perforation tout en étant parfaitement adapté pour être guidé sur une broche rigide jusqu'au contact osseux. L'invention a encore pour objet un dispositif permettant, au choix du praticien, de débuter une procédure opératoire manuellement puis de la terminer au moyen d'un entraînement automatique en rotation, telle qu'une perceuse, si la dureté de l'os le nécessite, et ce sans perte du point d'entrée dans l'os.

Le dispositif selon la présente invention est définit par la revendication 1. Les modes de réalisation préférés sont définis dans les revendications dépendantes.

Le dispositif selon la présente invention, du type trocart perforant, comprend une gaine externe comportant un tube rigide et un mandrin comportant une tige. La tige est adaptée pour coulisser dans la gaine externe et comporte à son extrémité distale une pointe perforante.

Préférentiellement, la pointe perforante est formée par un affûtage à biseaux de l'extrémité distale de la tige.

Avantageusement, l'extrémité distale de la tige comprend au moins une arête de coupe s'étendant depuis la pointe perforante jusqu'aux bords de la tige.

Suivant une caractéristique de l'invention, l'extrémité distale du tube comprend au moins deux segments à bord coupant de forme hélicoïdale.

De manière avantageuse, la gaine externe comprend en outre un raccord à l'extrémité proximale du tube, et le mandrin comprend en outre un bouchon à l'extrémité proximale de la tige. Le bouchon est adapté pour recevoir le raccord afin de former un ensemble solidarisé. Le raccord est perforé pour permettre le coulissage de la tige, et le bouchon est perforé pour permettre le coulissage de la broche guide.

Selon des modes de réalisation, l'ensemble formé de la gaine externe et du mandrin est monté dans une poignée ou dans un moyen automatique d'entraînement en rotation. Par exemple, le moyen automatique d'entraînement en rotation peut être une perceuse ayant un embout dans lequel est monté l'ensemble.

Avantageusement, le moyen automatique d'entraînement peut être pourvu d'une enveloppe protectrice. L'enveloppe protectrice peut, par exemple, être attachée à l'embout d'une perceuse au moyen d'un raccord qui est simultanément adapté à recevoir la gaine externe du trocart perforant.

À titre d'exemple, dans le cas d'une lésion osseuse siégeant sous une corticale dense, le praticien, après avoir procédé à une anesthésie locale suivant les techniques classiques, introduit la broche guide parallèlement ou au travers de l'aiguille anesthésique en place jusqu'à venir au contact de l'os. L'aiguille anesthésique est ensuite enlevée tout en laissant la broche guide en place. Le dispositif selon l'invention, équipé d'une poignée amovible, est introduit au travers des tissus jusqu'au contact de l'os tout en étant guidé sur broche. La broche est enlevée une fois la surface de la corticale atteinte, puis le praticien fore l'os en tournant manuellement le trocart sans perte du point d'entrée. Dans 80% à 90% des cas le forage se fera manuellement, mais si la paroi osseuse est très dure, le praticien peut enlever la poignée amovible et raccorder le trocart à une perceuse pour terminer la procédure opératoire.

La simplicité de la structure du dispositif de l'invention permet de proposer à l'utilisateur, en fonction de la profondeur ou de la localisation de la lésion, un instrument unique permettant au choix une voie directe ou bien guidée sur broche, et dont le forage peut être manuel ou à la perceuse, ou bien encore commencé manuellement pour être terminé à la perceuse. La combinaison d'un forage manuel avec un forage à la perceuse permet de bénéficier à la fois d'une grande précision de l'acte opératoire et d'une grande puissance quelle que soit la dureté de l'os.

D'autres caractéristiques et avantages de la présente invention apparaîtront dans la description suivante, relative à une forme préférentielle de réalisation, en référence aux dessins annexés, qui représentent:
- Figure 1 : vue en perspective d'un dispositif monté dans une poignée ;
- Figures 2 à 3 : vues en perspective d'un tube du dispositif selon l'invention monté dans un raccord, formant une gaine externe;
- Figures 4 à 5 : vues en perspective d'une tige rainurée d'un dispositif montée dans un bouchon, formant un mandrin;
- Figures 6 à 9 : vues d'un exemple de l'extrémité distale de la tige rainurée;
- Figures 10 et 11 : vues d'un exemple de l'extrémité distale de la tige d'un dispositif selon l'invention pourvue d'un trou;
- Figure 12 : vue en perspective d'une poignée apte à recevoir l'ensemble formé de la gaine et du mandrin du dispositif selon l'invention; et
- Figure 13: vue en perspective d'une perceuse apte à recevoir l'ensemble formé de la gaine et du mandrin du dispositif selon l'invention.

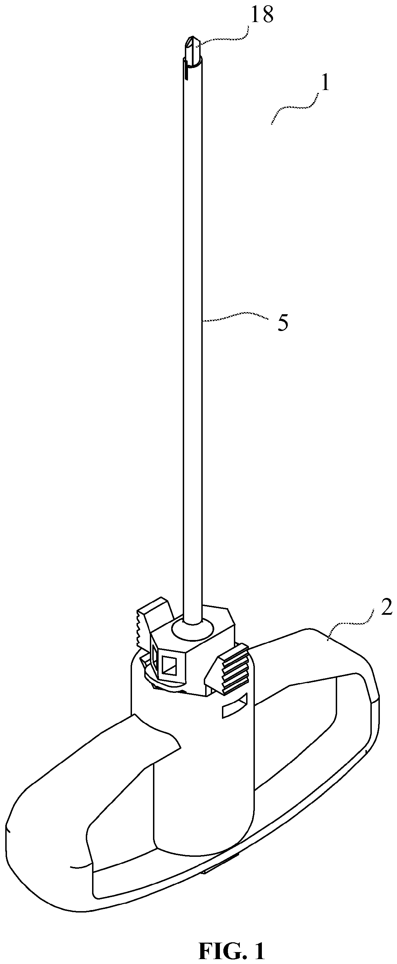

Le trocart 1, représentée sur la Figure 1, est composé d'une gaine externe 5 dans laquelle est monté un mandrin (dont seulement l'extrémité distale de la tige 18 est visible sur la Figure 1), et d'une poignée 2 dans laquelle l'ensemble gaine-mandrin est inséré.

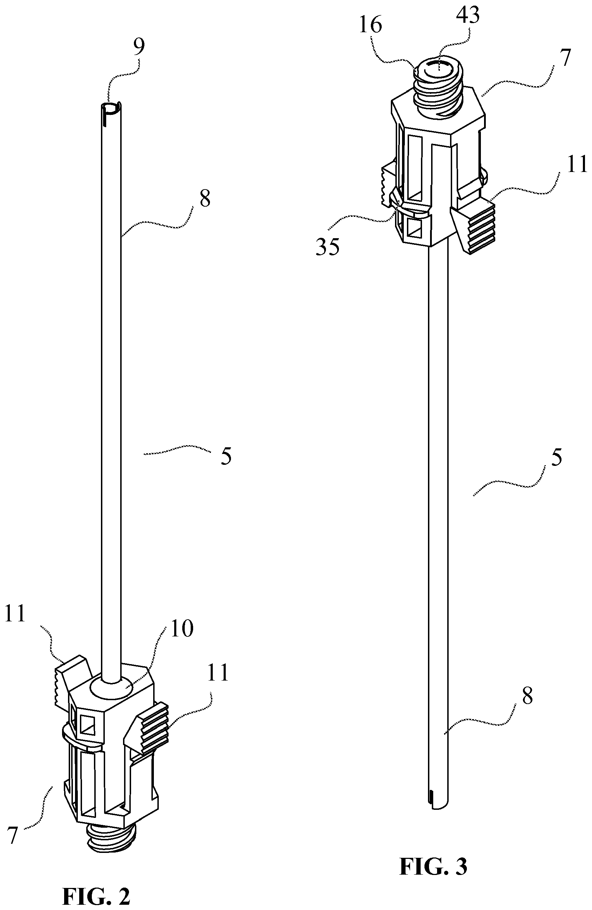

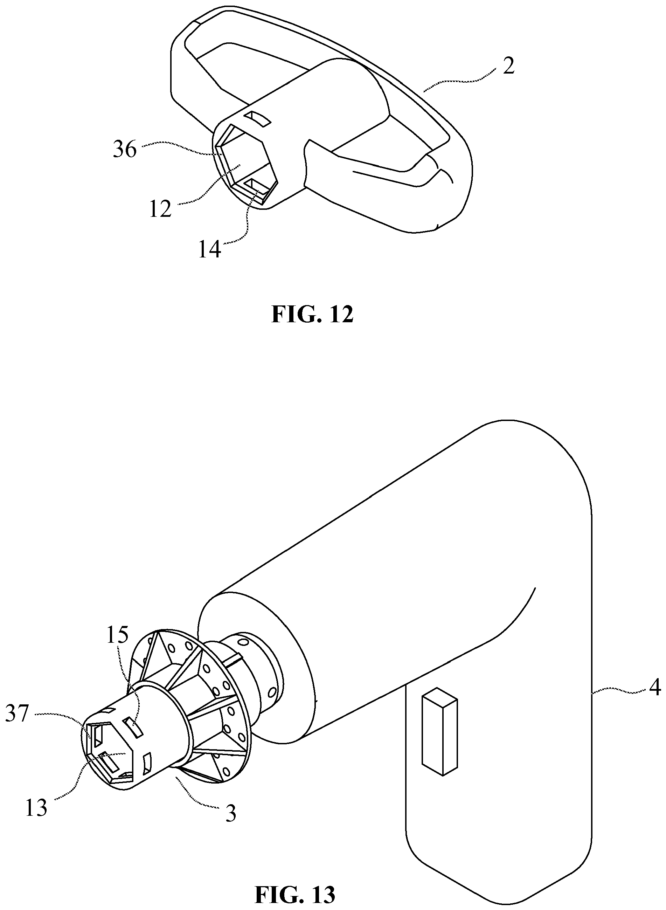

La gaine externe 5, représentée sur les Figures 2 et 3, comporte un raccord 7 dans lequel est logé le tube affûté 8. Le tube 8 comporte une extrémité distale 9 divisée en deux segments à bord coupant hélicoïdal tel que décrit dans la demande WO 2006/061514. Le raccord 7 est de forme hexagonale coopérant avec des cavités hexagonales 12 de la poignée 2 et des cavités hexagonales 13 de l'embout 3 d'une perceuse 4 (voir également les Figures 12 et 13). Le raccord 7 comporte un perçage 10, dans lequel le tube 8 est logé, et deux parties flexibles 11 s'encliquetant dans des évidements 14 de la poignée 2 ou dans des évidements 15 de l'embout 3 de la perceuse 4.

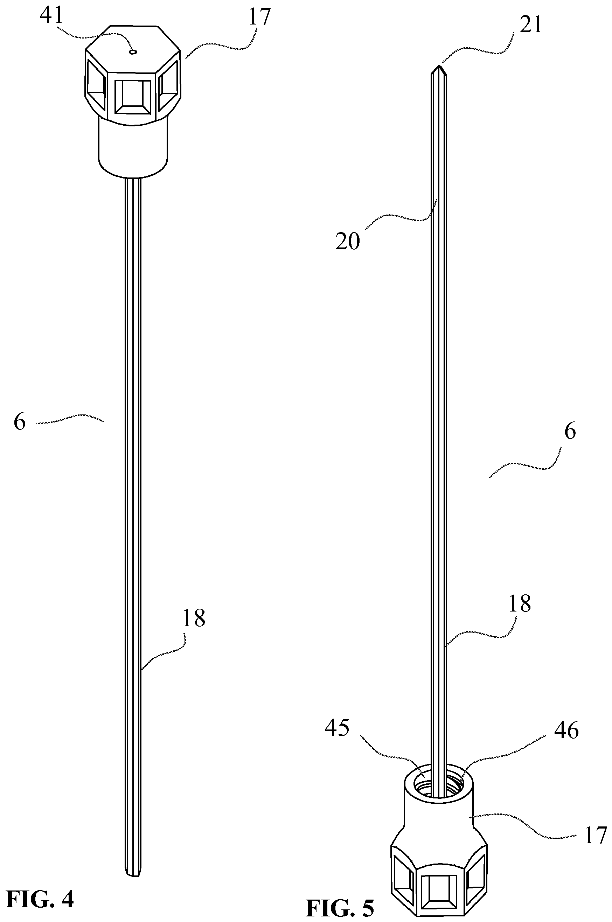

Le mandrin 6, représenté sur les Figures 4 et 5, comprend une tige rainurée 18 logée dans un bouchon 17. La tige rainurée 18 comporte une rainure longitudinale 20 en forme de V et une extrémité distale de forme pyramidale. Le bouchon 17 est de forme hexagonale coopérant avec les cavités hexagonales 12 de la poignée 2 et les cavités hexagonales 13 de l'embout 3 de la perceuse 4. Le bouchon 17 comporte un perçage 45 dans lequel la tige rainurée 18 est logée et un perçage 41 laissant passer la broche guide.

Le raccord 7 de la gaine externe 5 est entièrement perforé 43 de façon à permettre le coulissage de la tige 18. L'extrémité 16 de la gaine externe 5 de type embout Luer permet le vissage du bouchon 17. Le bouchon 17 comporte un filetage 46 coopérant avec le filetage 16 du raccord 7 permettant son vissage afin d'assembler la gaine externe 5 et le mandrin 6.

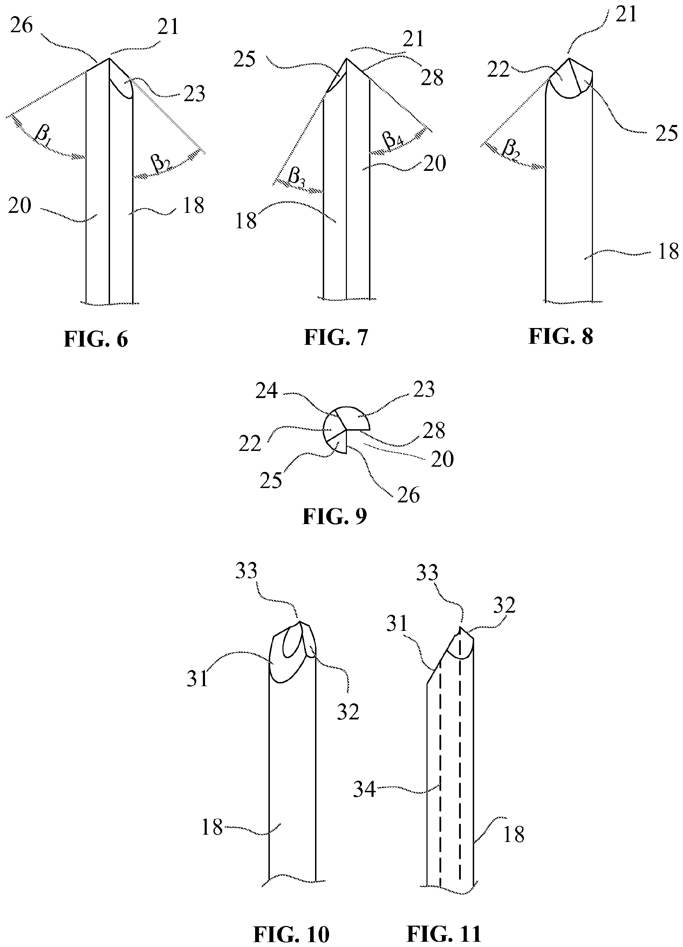

Les Figures 6 à 9 représentent des vues d'un exemple de l'extrémité distale de la tige 18. L'extrémité distale de la tige 18 comprend une pointe 21 ayant un affûtage pyramidal à trois biseaux. Deux des biseaux 22, 23 ont la même inclinaison (β₃), préférentiellement β₃ = 30° par rapport à l'axe de la tige 18, et s'étendent sur environ 120° de la section de la tige 18, générant une arête de coupe 24. Le troisième biseau 25, incliné préférentiellement suivant β₁ et à 40° par rapport à l'axe de la tige 18, génère une deuxième arête de coupe 26. La deuxième arête de coupe 26 est située sur l'une des faces de la rainure 20 et a une inclinaison β₁ plus importante que l'inclinaison β₂ de l'arête 24 par rapport à l'axe de la tige 18. Ceci permet d'avoir une meilleure coupe de l'os car la deuxième arête de coupe 26 bénéficie d'un angle d'attaque plus important, généré par l'une des faces de la rainure 20. L'inclinaison β₁ de l'arête de coupe 26 est plus importante que l'inclinaison β₂ de l'arête de coupe 24 et que l'inclinaison β₄ de l'autre arête de coupe 28 située sur l'autre face de la rainure longitudinale. L'intersection des trois biseaux génère la pointe 21 permettant au mandrin 6 de ne pas glisser sur l'os.

Les vues des Figures 10 et 11 montrent une extrémité distale de la tige 18 selon une variante. La tige 18 comporte, centré sur son axe, un trou 34 permettant le guidage sur broche. L'extrémité distale de la tige 18 comprend un affûtage pyramidal à trois biseaux de même inclinaison dont un biseau 31 est plus grand que les deux autres biseaux 32 de façon à générer une pointe 33 située sur le bord du trou 34.

La vue en perspective de la Figure 12 montre la poignée 2 comportant une cavité hexagonale 12 dans laquelle se monte la gaine externe 5 équipée du mandrin 6. Les parties flexibles 11 du raccord 7 s'encliquettent dans les évidements 14 pourvus à cet effet.

La vue en perspective de la Figure 13 montre une perceuse 4 sur laquelle est monté l'embout 3 comportant une cavité hexagonale 13, permettant le montage de la gaine externe 5 équipée du mandrin 6, et des évidements 15 dans lesquels s'encliquettent les parties flexibles 11 du raccord 7.

En se référant aux Figures 2, 3, 12 et 13, le chanfrein 35 du raccord 7 de la gaine externe 5 coopère avec les chanfreins 36 de la poignée 2 et les chanfreins 37 de l'embout 3 de la perceuse 4 de façon à réduire les jeux lors du forage de l'os.

## Revendications

1. Dispositif chirurgical, notamment pour la chirurgie et la radiologie interventionnelle, comprenant:
- une gaine externe (5) comportant un tube rigide (8); et
- un mandrin (6) comportant une tige (18), la tige (18) étant adaptée pour coulisser dans la gaine extérieur (5) et comportant à son extrémité distale, une pointe perforant (34),
dans lequel la tige (18) comporte, centré sur son axe, un trou (34) permettant le guidage sur broche dans lequel l'extrémité distale de la tige (18) comprend un affûtage pyramidal à trois biseaux de même inclinaison dont un biseau (31) est plus grand que les deux autres biseaux (32) de façon à générer une pointe (33) située sur le bord du trou (34).

2. Dispositif selon la revendication 1, dans lequel une extrémité distale du tube (8) comprend au moins deux segments à bord coupant hélicoïdal.

3. Dispositif selon l'une des revendications précédentes, dans lequel:
- la gaine externe (5) comprend en outre un raccord (7) à une extrémité proximale du tube (8), et
- le mandrin (6) comprend en outre un bouchon (17) à l'extrémité proximale de la tige (18),
- le bouchon (17) étant apte à recevoir le raccord (7) afin de former un ensemble solidarisé,
dans lequel le raccord (7) est perforée (43) pour permettre le coulissage de la tige (18), et le bouchon (17) est perforée (41) pour permettre le coulissage de la broche guide.

4. Dispositif selon la revendication 3, dans lequel l'ensemble formé de le gaine externe (5) et du mandrin (6) est monté dans une poignée (2) ou dans un moyen d'entraînement automatique en rotation (4).

5. Dispositif selon la revendication 4 dans lequel le bouchon (17) est de forme hexagonale coopérant avec un cavité hexagonales (12) de la poignée (2) ou un cavité hexagonales (13) de l'embout (3) de la perceuse (4).

6. Dispositif selon la revendication 5, dans lequel l'ensemble formé de le gaine externe (5) et du mandrin (6) est monté dans un moyen d'entraînement automatique en rotation (4).

7. Dispositif selon la revendication 5, dans lequel le bouchon (17) comporte un filetage (46) coopérant avec le filetage (16) du raccord (7).

8. Dispositif selon la revendication 7, dans lequel le filetage (16) du raccord (7) se présente sous la forme d'un connecteur luer.

## Patentansprüche

1. Chirurgische Vorrichtung, insbesondere für die Chirurgie und die interventionelle Radiologie, die Folgendes umfasst:
- eine Außenhülse (5), die ein starres Rohr (8) umfasst; und
- einen Dorn (6), der einen Stab (18) umfasst, wobei der Stab (18) zum Gleiten in der Außenhülse (5) ausgelegt ist und an seinem distalen Ende einen Perforationspunkt (34) aufweist,
wobei der Stab (18), auf seiner Achse zentriert, ein Loch (34) aufweist, das die Führung auf der Spindel zulässt, wobei das distale Ende des Stabs (18) pyramidenförmig mit drei Fasen derselben Neigung geschliffen ist, wobei eine Fase (31) größer ist als die beiden anderen Fasen (32), um eine Spitze (33) zu erzeugen, die sich am Rand des Lochs (34) befindet.

2. Vorrichtung nach Anspruch 1, bei der ein distales Ende des Rohrs (8) wenigstens zwei Segmente mit spiralförmiger Schneide umfasst.

3. Vorrichtung nach einem der vorherigen Ansprüche, bei der:
- die Außenhülse (5) ferner ein Anschlussstück (7) an einem proximalen Ende des Rohrs (8) aufweist, und
- der Dorn (6) ferner einen Stöpsel (17) am proximalen Ende des Stabs (18) umfasst,
- wobei der Stöpsel (17) das Anschlussstück (7) aufnehmen kann, um eine solidarisierte Baugruppe zu bilden,
wobei das Anschlussstück (7) perforiert (43) ist, um das Gleiten des Stabs (18) zuzulassen, und der Stöpsel (17) perforiert (41) ist, um das Gleiten der Führungsspindel zuzulassen.

4. Vorrichtung nach Anspruch 3, wobei die aus der Außenhülse (5) und dem Dorn (6) gebildete Baugruppe in einem Griff (2) oder einem automatischen Drehantriebsmittel (4) montiert ist.

5. Vorrichtung nach Anspruch 4, bei der der Stöpsel (17) eine hexagonale Form hat, die mit einem hexagonalen Hohlraum (12) des Griffs (2) oder einem hexagonalen Hohlraum (13) des Ansatzes (3) der Bohrmaschine (4) zusammenwirkt.

6. Vorrichtung nach Anspruch 5, bei der die aus der Außenhülse (5) und dem Dorn (6) gebildete Baugruppe in einem automatischen Drehantriebsmittel (4) montiert ist.

7. Vorrichtung nach Anspruch 5, bei der der Stöpsel (17) ein Gewinde (46) aufweist, das mit dem Gewinde (16) des Anschlussstücks (7) zusammenwirkt.

8. Vorrichtung nach Anspruch 7, bei der das Gewinde (16) des Anschlussstücks (7) in Form eines Luer-Anschlusses vorliegt.

## Claims

1. Surgical device, in particular for surgery and interventional radiology, comprising
- an outer sheath (5) having a rigid tube (8); and
- a mandrin (6) having a shaft (18), the shaft (18) being adapted to slide in the outer sheath (5) and having at its distal end a perforating tip (34),
wherein the shaft (18) has, centred on its axis, a hole (34) allowing on-pin guidance, wherein the distal end of the shaft (18) comprises pyramid tapering with three bevels of the same inclination, one bevel (31) of which is larger than the two other bevels (32) so as to produce a tip (33) located on the edge of the hole (34).

2. Device as claimed in claim 1, wherein a distal end of the tube (8) comprises at least two segments with a helicoidal cutting edge.

3. Device as claimed in any one of the preceding claims, wherein:
- the outer sheath (5) further comprises a connector (7) to a proximal end of the tube (8), and
- the mandrin (6) further comprises a plug (17) at the proximal end of the shaft (18),
- the plug (17) being adapted to receive the connector (7) so as to form an integrated assembly,
wherein the connector (7) is perforated (43) to allow sliding of the shaft (18), and the plug (17) is perforated (41) to allow sliding of the guide pin.

4. Device as claimed in claim 3, wherein the assembly formed by the outer sheath (5) and the mandrin (6) is mounted in a handle (2) or in an automatic rotational driving means (4).

5. Device as claimed in claim 4, wherein the plug (17) is hexagonal, co-operating with a hexagonal cavity (12) in the handle (2) or a hexagonal cavity (13) in the tip (3) of the drill (4).

6. Device as claimed in claim 5, wherein the assembly formed by the outer sheath (5) and the mandrin (6) is mounted in an automatic rotational driving means (4).

7. Device as claimed in claim 5, wherein the plug (17) has a thread (46) co-operating with the thread (16) of the connector (7).

8. Device as claimed in claim 7, wherein the thread (16) of the connector (7) is in the form of a Luer connector.
